# EUROPÄISCHE PATENTSCHRIFT

(11) **EP 1 148 910 B1**
(45) Veröffentlichungstag und Bekanntmachung des Hinweises auf die Patenterteilung: **12.10.2005**
(21) Anmeldenummer: 00976051.3
(22) Anmeldetag: 20.11.2000
(51) Int. Cl.: A61N 1/375

(54) **FILTERDURCHFÜHRUNG**
FILTER FEEDTHROUGH
TRAVERSEE FILTRANTE

(30) Priorität: 20.11.1999 DE 19957189
(43) Veröffentlichungstag der Anmeldung: 31.10.2001
(73) Patentinhaber: BIOTRONIK Mess- und Therapiegeräte GmbH & Co Ingenieurbüro Berlin, 12359 Berlin (DE)
(72) Erfinder: ECK, Stefan, 91315 Höchstadt (DE); FRAUENSTEIN, Boris, 91074 Herzogenaurach (DE); SCHALDACH, Max, verstorben (DE)
(74) Vertreter: Eisenführ, Speiser & Partner
(86) Internationale Anmeldenummer: PCT/EP2000/011518
(87) Internationale Veröffentlichungsnummer: WO 2001/037929

(56) Entgegenhaltungen:
- EP-A- 0 331 959
- EP-A- 0 916 364
- DE-A- 19 819 797
- US-A- 5 333 095
- US-A- 5 650 759

## Beschreibung

Die Erfindung betrifft eine uni- oder multipolare elektrische Filterdurchführung für ein implantierbares elektronisches Therapiegerät, beispielsweise einen Herzschrittmacher. Die Durchführung ist zum Einsetzen in eine Öffnung in einem Gehäuse des Therapiegerätes ausgebildet und weist voneinander galvanisch getrennt angeordnete Kontaktelemente sowie ein Befestigungsmittel zur Umbänderung mit einer Wandung des Gehäuses umfassenden Durchführungskörper und Filtermittel auf, welche einerseits mit jeweils einem der Kontaktelemente und andererseits jeweils mit dem ein Bezugspotential führenden Gehäuse elektrisch leitend verbunden sind. Die Filtermittel umfassen üblicherweise kapazitive Elemente. Die Kontaktelemente sind üblicherweise als stabförmige Pins ausgebildet.

Aus der amerikanischen Patentschrift US-PS 4 152 540 ist eine Filterdurchführung zur Verwendung bei einem elektronischen Herzschrittmacher bekannt, bei welcher ein Filterkondensator innerhalb einer Bohrung in der Isolationskeramik der Durchführung vorgesehen ist.

Des weiteren ist aus der europäischen Patentanmeldung EP 0 776 016 A2 eine Durchführung mit einer Kapazitäten aufweisenden, keramischen Filteranordnung für implantierbare Defibrillatoren und Herzschrittmacher bekannt. Die Filteranordnung ist als Schichtsystem aufgebaut und in die Durchführung integriert.

In einer aus der amerikanischen Patentschrift US-PS 5 650 016 bekannten Durchführung für implantierbare medizinische Therapiegeräte ist zur Integration eines als Chip-Kondensator oder als LC-Glied ausgebildeten Filtermittel vorgesehen, diese Bauteile mit der Durchführung zu verkapseln.

In der europäischen Anmeldung EP 0 916 364 wird eine Durchführung beschrieben, die dem Gegenstand dieser Anmeldung ähnelt. Dort werden jedoch die als Schichtkondensatoren ausgeführten Filtermittel direkt auf dem Durchführungskörper befestigt, wodurch diese den mechanischen Belastungen durch die durch das Verschweißen des Durchführungskörpers mit dem Gehäuse verursachten Spannungen und den irreversiblen Veränderungen der elektronischen Kennwerte der Filtermittel aufgrund der Hitzeentwicklung beim Schweißen ausgesetzt sind.

Die bekannten elektrischen Durchführungen weisen jedoch den Nachteil auf, dass die kapazitiven Filtermittel sowie die Kontaktierungsbereiche zwischen den elektrischen Pins und dem Bezugspotential gasdicht ausgeführt werden. Dies führt ― insbesondere unter Berücksichtigung der Vormontage der Filtermittel ― zu einem erheblichen Aufwand bei der Fertigung einer derartigen Durchführung und beim Einsetzen der Durchführung in das Gehäuse des zu implantierenden Therapiegeräts und dessen nachfolgender Prüfung auf Vakuumdichtigkeit.

Darüber hinaus sind die mit der Durchführung fest verbundenen Filtermittel in nachteiliger Weise einer hohen thermischen Belastung ausgesetzt, wenn die Durchführung in eine entsprechende Gehäuseöffnung des elektronischen Therapiegeräts eingeschweißt wird. Dies verlangt einerseits eine sehr feste mechanische Verbindung der Filtermittel an der Durchführung und führt andererseits häufig zu einer irreversiblen Veränderung der elektrischen Kennwerte der Filtermittel, was sich in meist nicht vorhersehbarem Maße störend auf die Betriebsführung des zu implantierenden Therapiegeräts auswirkt.

Ausgehend von den Mängeln des Standes der Technik liegt der Erfindung deshalb die Aufgabe zugrunde, eine uni- oder multipolare Durchführung der eingangs genannten Gattung anzugeben, welche besonders kostengünstig gefertigt und bei relativ geringer thermischer Belastung der Filtermittel derart montiert werden kann, dass darüber hinaus bei der Montage die Gefahr einer Veränderung der elektrischen Eigenschaften der Filtermittel weitestgehend ausgeschlossen ist.

Die Aufgabe wird durch eine Filterdurchführung der eingangs genannten Art gelöst, bei der die Filtermittel außerhalb des Durchführungskörpers angeordnet und derart mit ihm verbunden sind, dass sie im eingesetzten Zustand im Wesentlichen frei schwebend in das Innere des Gehäuses ragen.

Die Erfindung schließt die Erkenntnis ein, dass besondere Vorteile bei der Fertigung, der Montage und der mit einem Vakuum-Lecktest verbundenen Einbauprüfung einer Filtermittel aufweisenden uni- oder multipolaren elektrischen Durchführung nach Einbau in einem Gehäuse eines implantierbaren elektronischen Therapiegeräts zu erzielen sind, wenn für die Durchführung und die elektrischen Filtermittel eine räumliche Zuordnung gewählt ist, bei welcher eine Kontaktierung zwischen Filtermittel und Gehäusewandung sichergestellt ist und gleichzeitig die im Wesentlichen nicht vakuumdichte Konstruktion der verwendeten Filtermittel in günstige Weise außer Betracht bleiben kann. Hieraus ergibt sich der Vorteil, dass das eigentliche Dichtelement, das heißt, die Isolationskeramik und deren Verbindungen mit Flansch und Pins, unabhängig von den Filtermitteln hinsichtlich der Leckdichtigkeit getestet werden kann.

Erfindungsgemäß ist bei einer uni- oder multipolaren elektrischen Durchführung zum Einsetzen in eine Öffnung im Gehäuse eines implantierbaren elektronischen Therapiegeräts, mit einem in diese Öffnung einsetzbaren und ein, bevorzugt flanschförmiges, Befestigungsmittel zur Verbindung mit dem Gehäuse aufweisenden Durchführungskörper und mit als Kapazität ausgebildeten Filtermitteln, welche einerseits jeweils mit einem der voneinander galvanisch getrennt angeordneten elektrischen Pins der Durchführung und andererseits jeweils mit dem ein Bezugspotential führenden Gehäuse des elektronischen Therapiegeräts eine elektrisch leitende Verbindung aufweisen, vorgesehen, die Filtermittel unter Vermeidung einer körperlichen Integrität außerhalb der Durchführungskörpers anzuordnen. Die Filtermittel sind dabei derart mit dem Durchführungskörper verbunden, dass sie im Wesentlichen frei schwebend in das Innere des Gehäuses ragen.

Dadurch ergibt sich als wesentlicher Vorteil, dass an die Filtermittel keine Forderungen hinsichtlich einer Vakuumdichtigkeit gestellt werden müssen. Lediglich der Bereich des Gehäuses, an welchem die Durchführung mit ihrem Durchführungskörper in die Gehäusewandung eingesetzt wird, und der Durchführungskörper selbst sind vakuumdicht auszuführen, damit nach erfolgter Implantation des elektronischen Therapiegeräts keine Körperflüssigkeit in das Innere des Gehäuses eindringen kann.

Als weiterer Vorteil ergibt sich eine Entlastung hinsichtlich thermischer oder mechanischer Spannungen, so dass mechanische Beschädigungen insbesondere des Filters vermieden werden, welche nicht oder nur eingeschränkt detektiert werden können.

Entsprechend der bevorzugten Ausführungsform der Erfindung bilden die Filtermittel einen Filterblock, weicher mit dem Durchführungskörper der Durchführung auf gleicher Achse liegend angeordnet ist. Durch eine derartige Anordnung ergeben sich Vereinfachungen bei der Vormontage der erfindungsgemäßen Durchführung.

Das zur Verbindung der Durchführung mit Gehäuse des zu implantierenden Therapiegeräts an dem Durchführungskörper vorgesehene Befestigungsmittel ist bevorzugt als Ringflansch ausgebildet und weist einen sich in Richtung des Gehäuseinneren erstreckenden metallischen Kragen auf. An dessen freiem Ende ist der Filterblock unter Vermeidung einer körperlichen Integrität mit dem Durchführungskörper befestigt. Der Ringflansch bildet beim Einsetzen der Durchführung in eine Gehäuseöffnung den erforderlichen Anschlag, um die Durchführung mit einer vakuumdichten Schweißverbindung zwischen Durchführungskörper und Gehäusewandung zu fixieren.

Um eine möglichst spannungsfreie Verbindung zwischen dem Durchführungskörper und dem Filterblock herzustellen, welche auch nicht durch die bei dem Schweißvorgang freigesetzte Wärmemenge mechanisch unzulässig belastet wird, ist der metallische Kragen flexibel ausgebildet. Diese Flexibilität wird entsprechend der bevorzugten Ausführungsform der Erfindung auf einfache Weise dadurch erreicht, indem der metallische Kragen aus einem eine Lamellenstruktur aufweisenden Band gebildet ist. Die Lamellen sind an dem Kragen im Wesentlichen gleichmäßig verteilt angeordnet, so dass der Kragen im Wesentlichen die Form einer Krone aufweist.

Bei der Vormontage der Durchführung wird der zylindrisch ausgebildete Filterblock in den von dem kronenförmigen Kragen begrenzten Raum eingesetzt. Dabei ist vorgesehen, dass sich der Mantel des Filterblocks auf der Innenseite der Lamellen des Kragens abstützt.

Durch eine Kontaktierung der Lamellen an dem Mantel des Filterblocks wird eine galvanische Verbindung zwischen den einzelnen Pins der Durchführung zugeordneten Filterelementen und dem das Bezugspotential führenden Gehäuse des implantierbaren elektronischen Therapiegeräts hergestellt.

Der Filterblock weist eine Mehrzahl von keramischen Scheiben auf, welche in Schichtung übereinander angeordnet sind. Dabei ist eine der Anzahl der elektrischen Pins entsprechende Anzahl von als metallisiertes Substrat ausgebildeter Scheiben vorgesehen, welche zur Bildung der kapazitiven Filterelemente des Filterblocks jeweils zwischen zwei nichtmetallisierten keramischen Schichten angeordnet sind.

Zur galvanischen Verbindung der Filterelemente mit der das Bezugspotential führenden Gehäusewandung ist es ausreichend, wenn die als metallisiertes Substrat ausgebildeten Keramikscheiben jeweils mit nur einer der an dem kronenförmigen Kragen vorgesehenen Lamelle kontaktiert sind.

Nach einer weiteren Variante der Erfindung sind für die multipolare elektrische Durchführung vier elektrische Pins vorgesehen, so dass der Filterblock insgesamt vier als beschichtetes Substrat ausgebildete und vier beschichtungsfreie keramische Scheiben aufweist, welche - wechselweise übereinander geschichtet -zu dem Filterblock zusammengefaßt sind.

Nach einer vorteilhaften Weiterbildung der Erfindung ist an dem Kragen ein zusätzlicher Anschlusspin zur galvanischen Verbindung mit der Signalerzeugungs- und -verarbeitungseinrichtung des elektronischen Therapiegeräts vorgesehen. Damit kann auf einfache Weise auch diese Einrichtung mit dem Bezugpotential verbunden werden.

In einer vorteilhaften Variante der Erfindung ist dieser Anschluß bandförmig ausgebildet, wodurch sich besondere Vereinfachungen bei der Vormontage der Signalerzeugungs- und -verarbeitungseinrichtung ergeben.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt.

Es zeigen:
- Figur 1: eine bevorzugte Ausführungsform der Erfindung in perspektivischer Darstellung,
- Figur 2: die in Figur 1 gezeigte Ausführungsform der Erfindung in Teilschnitt- ansicht von der Seite,
- Figur 3: die in Figur gezeigte Ausführungsform in Ansicht von unten sowie
- Figur 4: die Darstellung der Ansicht eines Schnittes längs der Linie A...A gemäß Figur 1,
- Figur 5: eine Darstellung einer alternativen Ausführungsform als Schnittansicht längs der Linie A...A gemäß Figur 1

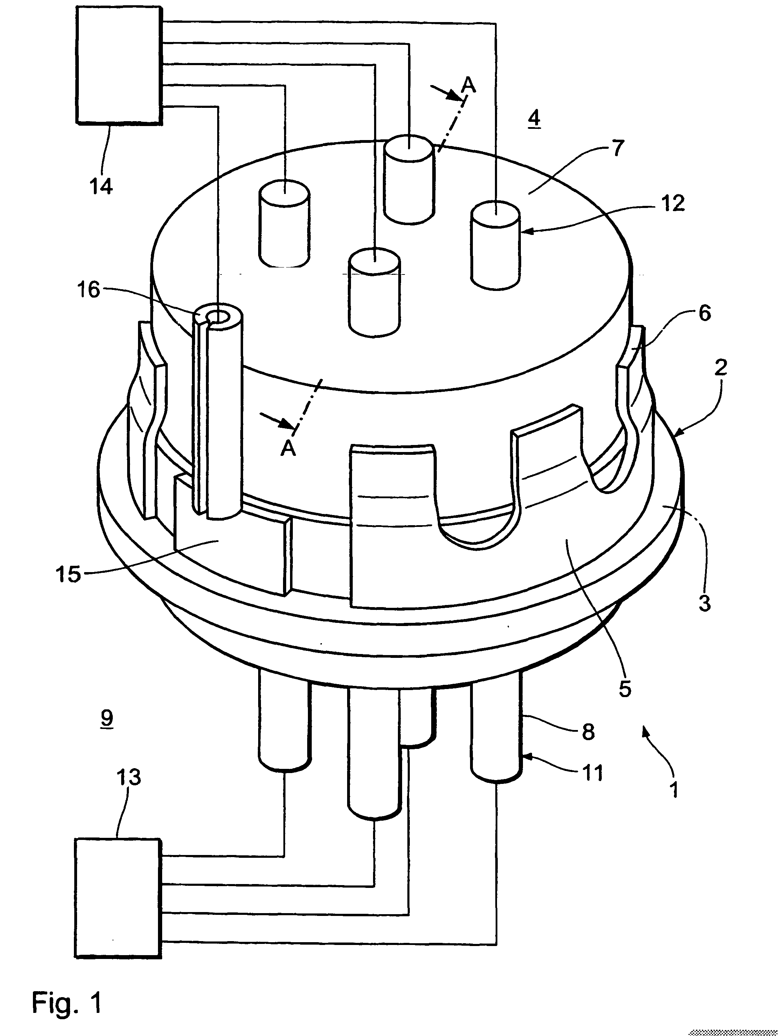

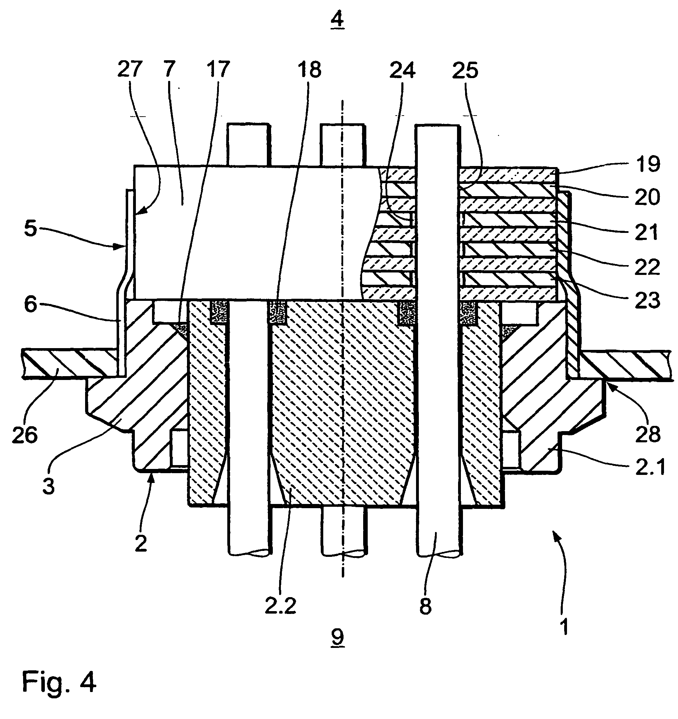

Die in Figur 1 gezeigte multipolare elektrische Durchführung 1 weist einen im Wesentlichen zylindrisch ausgebildeten Durchführungskörper 2 auf, welcher einen ringförmigen Flansch 3 trägt. An diesem Flansch ist ein in Richtung des Innenraums 4 eines (nicht dargestellten) Gehäuses (vergleiche die Position 4 in Figur 4) eines implantierbaren Therapiegeräts weisender Kragen 5 befestigt, welcher an seinem freien Ende in einzelne Lamellen 6 ausläuft. Die Lamellen 6 sind in Form und Größe gleichartig ausgebildet und gleichmäßig am Umfang des Kragens verteilt angeordnet. Der Kragen weist dadurch die Form einer Krone auf, welche in ihrem Lamellenbereich besonders flexibel ist.

Der Innenbereich dieser Krone wird von dem aus mehreren kapazitiven Filterelementen (vergleiche die Positionen 19 bis 23 in Figur 4) gebildeten Filterblock 7 ausgefüllt, welcher außenseitig mit den einzelnen Lamellen 6 galvanisch verbunden ist.

Elektrische Kontaktelemente bildende Pins 8 erstrecken sich von einem headerseitigen Anschlussbereich 9 der Durchführung 1 bis in den Gehäuseinnenraum 4 und durchdringen dabei sowohl den Durchführungskörper 2 und als auch den Filterblock 7. Die Art der Kontaktierung der Pins innerhalb des Filterblocks 7 der Durchführung 1 ist in Figur 4 gezeigt. Die Enden 11, 12 der Pins 8 sind mit einem Header 10 bzw. einer Signalerzeugungs- und -verarbeitungseinrichtung 14 des implantierbaren elektronischen Therapiegeräts verbunden.

An dem Kragen 5 ist eine zusätzliche, mittels einer Befestigungslasche 15 verbundene Kontaktfahne 16 vorgesehen, über welche die Signalerzeugungs- und -verarbeitungeinrichtung 14 auf einfache Weise mit der Wandung des das Bezugspotential führenden Gehäuses 26 des implantierbaren elektrischen Therapiegeräts verbunden werden kann. Dadurch sind Vereinfachungen bei der Montage der multipolaren Durchführungen möglich.

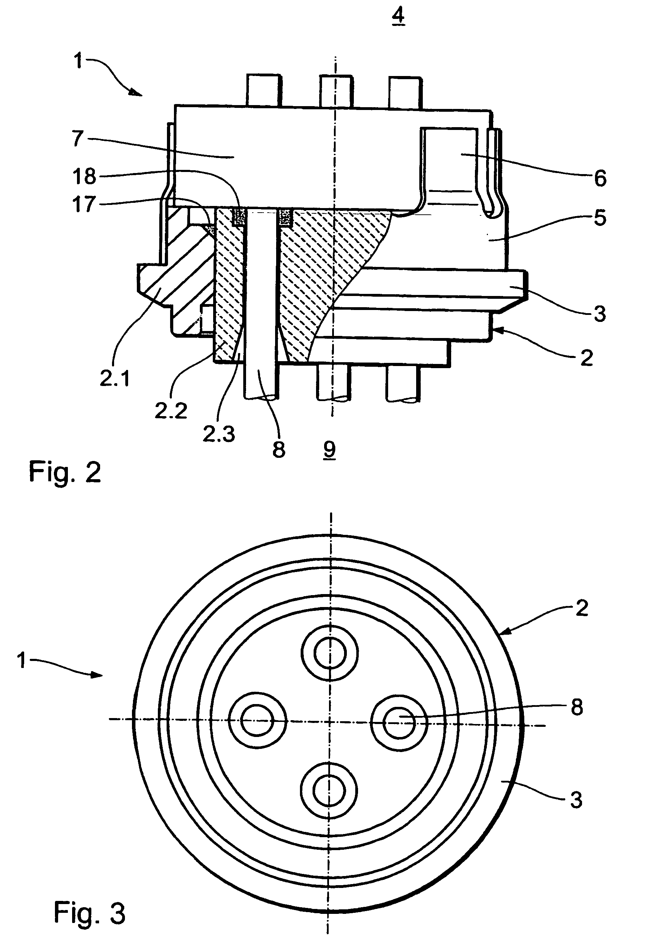

In den Figuren 2 und 3 ist die Form des Durchführungskörpers 2 der multipolaren elektrischen Durchführung 1 im Detail dargestellt.

Der Durchführungskörper 2 besteht aus einer im Wesentlichen zylindrisch ausgebildeten Hülse 2.1, an welche der Ringflansch 3 angeformt ist, und einem keramischen Einsatz 2.2, welcher den Innenraum der Hülse vollständig ausfüllt. Die einander berührenden Mantelflächen von Hülse 2.1 und keramischem Einsatz 2.2 sind durch ein dichtendes Fügemittel 17 wie Lot, Klebemasse oder, wie im konkreten Fall bevorzugt, Gold vakuumdicht miteinander verbunden. Die Pins 8 sind in Bohrungen durch diesen keramischen Einsatz vakuumdicht geführt. Die die entsprechenden Mantelflächen verbindende Klebemasse ist mit 18 bezeichnet.

Die trichterförmige Erweiterung 2.3 der im keramischen Einsatz 2.2 vorgesehenen Bohrungen für die Pins 8 auf der Headerseite 9 der Durchführung 1 ermöglicht im geringen Maße eine radiale Beweglichkeit der aus dem keramischen Einsatz herausragenden Enden der hier fixierten Pins 8, so dass sich bei der Montage des Headers (vergleiche die Position 13 in Figur 1) nach der Plazierung der Durchführung 1 im Gehäuse des implantierbaren elektrischen Therapiegeräts Vereinfachungen ergeben. Hauptzweck der trichterförmigen Erweiterungen oder Kegelsenkungen 2.3 ist die elektrische Isolation der Pins und insbesondere der Pin-Flansche voneinander, in dem Leckströme auf der Keramikoberfläche vermieden werden.

Figur 4 zeigt den Filterblock 7 der multipolaren elektrischen Durchführung 1 in einer schematisierten Teilschnittdarstellung.

Der Filterblock 7 weist eine Mehrzahl übereinanderliegend angeordneten keramischer Scheiben 19, 20, 21, 22, 23 auf, von denen die Scheiben 20, 21, 22 und 23 als metallisiertes Substrat ausgebildet sind. Die Scheiben 19 sind jeweils gleichartig ausgebildet und zwischen zwei eine Metallisierung aufweisenden Schichten angeordnet. Dieser Schichtaufbau, bestehend aus abwechselnd aufeinander folgenden Keramikscheiben und eine Metallisierung aufweisenden Schichten wird vorzugsweise durch Keramikscheiben erzielt, die jeweils in geeigneter Weise metallisiert sind. In den Keramikscheiben 19 sind vier gleich große Bohrungen zur Führung der Pins 8 vorgesehen. Die Scheiben 20, 21, 22 und 23 bilden paarweise einen Kondensator, wobei die zwischen ihnen befindliche,nicht metallisierte Keramikscheibe 19 als Dielektrikum dient. Die als metallisiertes Substrat ausgebildeten Scheiben 20, 21, 22, 23 sind ebenfalls gleichartig ausgebildet und weisen jeweils eine galvanische Verbindung mit nur einem der Pins 8 auf. Dazu sind in diesen Scheiben drei Bohrungen 24 vorgesehen, welche drei der sich durch den Filterblock 7 erstreckenden Pins beabstandet umgreifen. Der Durchmesser der vierten Bohrung ist geringer als der Bohrungen 24 und entspricht im Wesentlichen dem Außendurchmesser der Pins 8. An dieser Bohrung befindet sich jeweils die Kontaktierungsstelle 25 der Pins 8 mit der entsprechenden metallisierten Scheibe 20, 21, 22 oder 23. An ihrer Peripherie sind die Scheiben 20, 21, 22 und 23 jeweils mit den Lamellen 6 des Kragens 5 galvanisch verbunden und damit in Kontakt mit der Wandung 26 des das Bezugspotential (Massepotential) führenden Gehäuses des implantierbaren elektronischen Therapiegeräts. Die entsprechende Kontaktstelle ist mit 27 bezeichnet.

Durch die Anordnung von Lamellen 6 an seinem freien Ende weist der Kragen eine ausreichende Flexibilität auf, um mechanische Spannungen, welche durch Wärmeentwicklung beim Herstellen der Verbindung zwischen der Gehäusewandung 26 und der Hülse 2.1 des Durchführungskörpers 2 entstehen, aufzunehmen, ohne dass der Filterblock mechanisch belastet wird. Durch den relativ großen Abstand des über dem Durchführungskörper 2 schwebend positionierten Filterblock 7 zu der Schweißstelle 28 führt die Wärmeentwicklung beim Einschweißen der Durchführung 1 in das Gehäuse 26 des implantierbaren Therapiegeräts nicht zu einer solchen thermischen Belastung der einzelnen Filterelemente des Filterblocks 7, dass sich deren elektrische Werte irreversibel ändern.

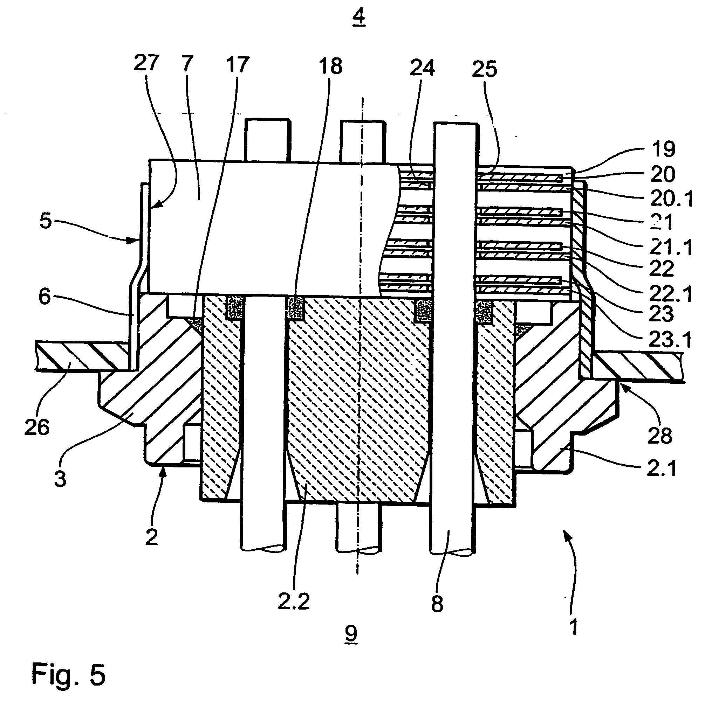

Die in Figur 5 dargestellte Ausführungsform der multipolaren elektrischen Durchführung 1 unterscheidet sich von der in Figur 4 dargestellten Ausführungsform im Wesentlichen durch den Aufbau des Filterblocks 7.

Zusätzlich zu den Scheiben 20, 21, 22 und 23, von denen jeweils eine mit einem der Pins 8 verbunden ist, weist der Filterblock 7 aus Figur 5 vier von metallisierten Substrat gebildete, elektrisch leitende Scheiben 20.1, 21.1, 22.1 und 23.1 auf, die an ihrer peripheren Kante elektrisch leitend mit den Lamellen 6 des Kragens 5 verbunden sind. Im Unterschied zu Figur 4 sind von den Scheiben 20, 21, 22 und 23 jeweils eine mit genau einem der Pins 8 verbunden, nicht jedoch mit den Lamellen 6 des Kragens 5. Die Scheiben 20, 21, 22 und 23 sowie die Scheiben 20.1, 21.1, 22.1 und 23.1 sind einander paarweise gegenüberliegend angeordnet, und zwar die Scheibe 20 der Scheibe 20.1, die Scheibe 21 der Scheibe 21.1, die Scheibe 22 der Scheibe 22.1 und die Scheibe 23 der Scheibe 23.1.

Von den einander gegenüberliegenden Scheiben eines jeweiligen Scheibenpaares ist somit eine der Scheiben mit einem der Pins 8 verbunden, während die jeweils andere Scheibe eines Paares mit dem Kragen 5 galvanisch verbunden ist. Jedes Scheibenpaar 20, 20.1 und 21, 21.1 und 22, 22.1 sowie 23, 23.1 bildet einen Kondensator, der zwischen jeweils genau einen der Pins 8 und den Kragen 5 geschaltet ist.

Die Bohrungen 24 in den Scheiben 20, 20.1, 21, 21.1, 22, 22.1, 23 und 23.1 sind so ausgeführt, dass nur jeweils genau eine Bohrung 24 in den Scheiben 20, 21, 22 und 23 so eng ausgeführt ist, dass die jeweilige der Scheiben 20, 21, 22 und 23 den entsprechenden Pin 8 kontaktiert, während alle übrigen Bohrungen, insbesondere sämtliche Bohrungen 24 in den Scheiben 20.1, 21.1, 22.1 und 23.1 einen größeren Durchmesser aufweisen, so dass diese Scheiben einen Abstand zu dem jeweiligen Pin 8 haben und diesen nicht elektrisch kontaktieren.

In den Figuren 1 bis 5 sind jeweils multipolare elektrische Durchführungen beschrieben.

In gleicher Weise, wie die beschriebene multipolaren Durchführungen lassen sich auch unipolare Filterdurchführungen realisieren. Diese weisen beispielsweise nur einen Pin von der Art der Pins 8 auf, der beispielsweise mit einer Reihe elektrisch leitender Scheiben wie den Scheiben 20, 21, 22 und 23 verbunden ist, während eine zweite Art Scheiben entsprechend den elektrisch leitenden Scheiben 20.1, 21.1, 22.1 und 23.1 wie zuvor beschrieben mit dem Kragen 5 elektrisch leitend verbunden ist. Die Erfindung beschränkt sich in ihrer Ausführung nicht auf die vorstehend angegebenen bevorzugten Ausführungsbeispiele. Vielmehr isteine Anzahl von Varianten möglich, welche von der dargestellten Lösung Gebrauch macht.

## Patentansprüche

1. Uni- oder multipolare elektrische Filterdurchführung (1) zum Einsetzen in eine Öffnung in einem Gehäuse (26) eines implantierbaren elektronischen Therapiegerätes, die einen in diese Öffnung einsetzbaren Durchführungskörper (2) mit einem oder mehreren voneinander galvanisch getrennt angeordneten Kontaktelementen (8) sowie ein Befestigungsmittel (3) zur Verbindung mit einer Wandung des Gehäuses (26), und, vorzugsweise als Kapazität ausgebildete, Filtermittel (7) umfaßt, welche einerseits jeweils mit einem der Kontaktelemente (8) und andererseits jeweils mit dem ein Bezugspotential führenden Befestigungsmittel (3) galvanisch verbunden sind wobei das Befestigungsmittel (3) des Durchführungskörpers (2) flanschartig ausgebildet ist, **daduch gekennzeichnet, dass** das Befestigungsmittel (3) einen in das Gehausemnere (4) weisenden, flexibel ausgebildeten, metallischen Kragen (5) mit Lammellen (6) an seinem freien Ende tragt, worin die Filtermittel (7) angeordnet und derart mit dem Durchführungskörper (2) verbunden sind, dass sie im eingesetzten Zustand im Wesentlichen frei schwebend über dem Durchführungskörper (2) positioniert sind.

2. Filterdurchführung nach Anspruch 1, **dadurch gekennzeichnet, dass** die Filtermittel einen Filterblock (7) bilden, welcher mit dem Durchführungskörper (2) auf gleicher Achse liegend angeordnet ist.

3. Filterdurchführung nach Anspruch 1 oder 2, **dadurch gekennzeichnet, dass** der Filterblock (7) an dem freien Ende des in das Gehäuseinnere (4) weisenden, metallischen Kragens (5) des Befestigungsmittels (3) des Durchführungskörpers (2) befestigt ist.

4. Filterdurchführung nach Anspruch 1, **dadurch gekennzeichnet, dass** die Lamellen (6) an dem Kragen (5) im Wesentlichen gleichmäßig verteilt angeordnet sind.

5. Filterdurchführung nach Anspruch 4, **dadurch gekennzeichnet, dass** der Kragen (5) die Form einer Krone aufweist.

6. Filterdurchführung nach Anspruch 1, **dadurch gekennzeichnet, dass** sich der Mantel des Filterblocks (7) auf der Innenseite der Lamellen (6) abstützt.

7. Filterdurchführung nach Anspruch 1, **dadurch gekennzeichnet, dass** der Filterblock (7) eine Mehrzahl keramischer, in Schichtung angeordneter Scheiben (19, 20, 20.1, 21, 21.1, 22, 22.1, 23, 23.1) aufweist, wobei eine der Anzahl der Kontaktelemente (8) entsprechende Anzahl der Scheiben (20, 21, 22, 23) als metallisiertes Substrat ausgebildet ist, welche jeweils zwischen zwei nichtmetallisierten keramischen Scheiben (19) angeordnet sind.

8. Filterdurchführung nach Anspruch 7, **dadurch gekennzeichnet, dass** von den als metallisiertes Substrat ausgebildeten Scheiben (20, 20.1, 21, 21.1, 22, 22.1, 23, 23.1) jeweils einige (20.1, 21.1, 22.1, 23.1) mit mindestens einer Lamelle (6) galvanisch verbunden sind.

9. Filterdurchführung nach Anspruch 8, **dadurch gekennzeichnet, dass** in dem Durchführungskörper (2) vier elektrische Pins (8) als Kontaktelemente vorgesehen sind.

10. Filterdurchführung nach Anspruch 9, **dadurch gekennzeichnet, dass** jeweils ein Pin (8) mit jeweils einer als metallisiertes Substrat ausgebildeten Scheibe (20, 21, 22, 23) elektrisch verbunden ist.

11. Filterdurchführung nach Anspruch 10, **dadurch gekennzeichnet, dass** die als metallisiertes Substrat ausgebildeten Scheiben (20, 20.1, 21, 21.1, 22, 22.1, 23, 23.1) eine der Anzahl der Pins (8) ensprechende Anzahl von Bohrungen (24) zur Aufnahme der Pins (8) aufweisen.

12. Filterdurchführung nach Anspruch 11, **dadurch gekennzeichnet, dass** die Bohrungen (24) in den als metallisiertes Substrat ausgebildeten Scheiben (20, 20.1, 21, 21.1, 22, 22.1, 23, 23.1) derart ausgebildet sind, dass diejenige Bohrung in einer der als metallisiertes Substrat ausgebildeten Scheiben (20, 21, 22, 23), die den jeweiligen Pin (8) umfaßt der mit der entsprechenden, als metallisiertes Substrat ausgebildeten Scheibe (20, 21, 22, 23) elektrisch verbunden ist, einen geringeren Durchmesser aufweist, als die übrigen Bohrungen (24).

13. Filterdurchführung nach Anspruch 1, **dadurch gekennzeichnet, dass** an dem Kragen (5) ein zusätzlicher Anschlusspin (16) zur galvanischen Verbindung einer Signalerzeugungs- und -verarbeitungseinrichtung (14) des implantierbaren elektronischen Therapiegerätes mit der andung des Gehäuses (26) vorgesehen ist.

14. Filterdurchführung nach Anspruch 13, **dadurch gekennzeichnet, dass** der Anschlusspin (16) im Wesentlichen bandförmig ausgebildet ist.

15. Implantierbares elektronisches Therapiegerät mit einem Gehäuse (26) und einer in diesem Gehäuse (26) angeordneten elektronischen Signalerzeugungs- und verarbeitungseinrichtung (14), **gekennzeichnet durch** eine uni- oder multipolare elektrische Filterdurchführung (1) nach einem der vorhergehenden Ansprüche zur Verbindung der elektronischen Signalerzeugungs- und verarbeitungseinrichtung (14) mit zum Betrieb des Therapiegerätes erforderlichen Signal- und Steuerleitungen.

## Claims

1. Unipolar or multipolar electrical filter feedthrough device (1) for insertion into an aperture in a housing (26) of an implantable electronic therapeutic device, the feedthrough device comprising a feedthrough body (2) that may be inserted into this aperture, with one or more mutually electrically separately arranged contact elements (8), and a fixing means (3) for connection to a wall of the housing (26) and filter means (7), preferably configured as a capacitor and each connected in an electrically conductive manner, on the one hand, to one of the contact elements (8) and, on the other hand, to the fixing means (3), the fixing means (3) of the feedthrough body (2) being of flange-like construction, **characterised in that** the fixing means (3) holds a flexible metal collar (5) pointing into the housing interior (4) and having lamellae at its free end, wherein the filter means are arranged and connected to the feedthrough body (2) such that, in the inserted state, they are positioned above the feedthrough body (2) in a substantially freely suspended manner.

2. Filter feedthrough device according to claim 1, **characterised in that** the filter means form a filter block (7) arranged on the same axis as the feedthrough body (2).

3. Filter feedthrough device according to either claim 1 or claim 2, **characterised in that** the filter block (7) is fastened to the free end of the metal collar (5) of the fixing means (3) of the feedthrough body (2), which collar points into the housing interior (4).

4. Filter feedthrough device according to claim 1, **characterised in that** the lamellae (6) are substantially uniformly distributed on the collar (5).

5. Filter feedthrough device according to claim 4, **characterised in that** the collar (5) has the shape of a crown.

6. Filter feedthrough device according to claim 1, **characterised in that** the shell of the filter block (7) rests on the inner side of the lamellae (6).

7. Filter feedthrough device according to claim 1, **characterised in that** the filter block comprises (7) a plurality of ceramic discs (19, 20, 20.1, 21, 21.1, 22, 22.1, 23, 23.1) arranged in layers, a number of discs (20, 21, 22, 23), corresponding to the number of contact elements (8), being configured as a metal-coated substrate and each being arranged between two non-metal-coated ceramic discs (19).

8. Filter feedthrough device according to claim 7, **characterised in that** some (20.1, 21.1, 22.1, 23.1) of the discs (20, 20.1, 21, 21.1, 22, 22.1, 23, 23.1) configured as a metal-coated substrate are connected in an electrically conductive manner to at least one lamella (6).

9. Filter feedthrough device according to claim 8, **characterised in that** four electrical pins (8) are provided as contact elements in the feedthrough body (2).

10. Filter feedthrough device according to claim 9, **characterised in that** one pin (8) is in each case connected in an electrically conductive manner to a respective disc (20, 21, 22, 23) configured as a metal-coated substrate.

11. Filter feedthrough device according to claim 10, **characterised in that**, for receiving the pins (8), the discs (20, 20.1, 21, 21.1, 22, 22.1, 23, 23.1) configured as a metal-coated substrate comprise a number of bores (24) corresponding to the number of pins (8).

12. Filter feedthrough device according to claim 11, **characterised in that** the bores (24) in the discs (20, 20.1, 21, 21.1, 22, 22.1, 23, 23.1) configured as a metal-coated substrate are formed such that the bore in one of the discs (20, 21, 22, 23) configured as a metal-coated substrate that contains the pin (8) that is electrically connected to the corresponding disc (20, 21, 22, 23) configured as a metal-coated substrate has a smaller diameter than the remaining bores (24).

13. Filter feedthrough device according to claim 1 **characterised in that** an additional connection pin (16) is provided on the collar (5) for electrical connection of a signal-generating and signal-processing device (14) of the implantable electronic therapeutic device to the wall of the housing (26).

14. Filter feedthrough device according to claim 13, **characterised in that** the connection pin (16) is substantially strip-shaped.

15. Implantable electronic therapeutic device comprising a housing (26) and an electronic signal-generating and signal-processing device arranged in this housing (26), **characterised by** a unipolar or multipolar electrical filter feedthrough device (1) according to any one of the preceding claims for connecting the electronic signal-generating and signal-processing device (14) to the signal and control lines required for the operation of the therapeutic device.

## Revendications

1. Traversée filtrante électrique unipolaire ou multipolaire (1) destinée à être insérée dans une ouverture d'un boîtier (26) d'un appareil de thérapie électronique pouvant être implanté, traversée qui comprend un bloc de traversée (2) pouvant être inséré dans cette ouverture avec un ou plusieurs éléments de contact (8) séparés galvaniquement les uns des autres ainsi qu'un moyen de fixation (3) pour raccordement à une paroi du boîtier (26) et des moyens de filtrage (7) réalisés de préférence sous forme de capacité, lesquels sont chacun galvaniquement reliés d'une part à l'un des éléments de contact (8) et d'autre part au moyen de fixation (3) conduisant un potentiel de référence, le moyen de fixation (3) du bloc de traversée (2) étant réalisé à mode de bride, **caractérisée en ce que** le moyen de fixation (3) porte, sur son extrémité libre, un collet métallique (5), réalisé de manière flexible, orienté vers l'intérieur du boîtier (6), avec des lamelles, dans laquelle les moyens de filtrage (7) sont disposés et reliés au bloc de traversée (2) de telle sorte qu'ils sont positionnés, en flottant librement pour l'essentiel, au-dessus du bloc de traversée (2) lorsqu'ils sont insérés.

2. Traversée filtrante selon la revendication 1, **caractérisée en ce que** les moyens de filtrage forment un bloc de filtrage (7), lequel est disposé de manière à reposer sur le même axe que le bloc de traversée (2).

3. Traversée filtrante selon la revendication 1 ou 2, **caractérisée en ce que** le bloc de filtrage (7) est fixé sur l'extrémité libre du collet métallique (5), orienté en direction de l'intérieur du boîtier (4), du moyen de fixation (3) du bloc de traversée (2).

4. Traversée filtrante selon la revendication 1, **caractérisée en ce que** les lamelles (6) sont disposées sur le collet (5) suivant une répartition homogène pour l'essentiel.

5. Traversée filtrante selon la revendication 4, **caractérisée en ce que** le collet (5) présente la forme d'une couronne.

6. Traversée filtrante selon la revendication 1, **caractérisée en ce que** l'enveloppe du bloc de filtrage (7) s'étaie sur la face interne des lamelles (6).

7. Traversée filtrante selon la revendication 1, **caractérisée en ce que** le bloc de filtrage (7) comporte une multitude de disques céramiques (19, 20, 20.1, 21, 21.1, 22, 22.1, 23, 23.1) disposés en stratification, un nombre de disques (20, 21, 22, 23) correspondant au nombre d'éléments de contact (8) étant réalisé sous forme de substrat métallisé, lesquels disques sont chacun disposés entre deux couches céramiques non métallisées (19).

8. Traversée filtrante selon la revendication 7, **caractérisée en ce que**, parmi les disques (20, 20.1, 21, 21.1, 22, 22.1, 23, 23.1) réalisés sous forme de substrat métallique, certains (20.1, 21.1, 22.1, 23.1) sont galvaniquement reliés à au moins une lamelle (6).

9. Traversée filtrante selon la revendication 8, **caractérisée en ce que** quatre broches électriques (8) sont prévues en tant qu'éléments de contact dans le bloc de traversée (2).

10. Traversée filtrante selon la revendication 9, **caractérisée en ce que** chaque broche (8) est électriquement reliée à un disque (20, 21, 22, 23) réalisé sous forme de substrat métallisé.

11. Traversée filtrante selon la revendication 10, **caractérisée en ce que** les disques (20, 20.1, 21, 21.1, 22, 22.1, 23, 23.1) réalisés sous forme de substrat métallique comportent un nombre de trous (24), pour loger les broches (8), correspondant au nombre de broches (8).

12. Traversée filtrante selon la revendication 11, **caractérisée en ce que** les trous (24) dans les disques (20, 20.1, 21, 21.1, 22, 22.1, 23, 23.1) réalisés sous forme de substrat métallique sont réalisés de telle sorte que le trou, dans l'un disques (20, 21, 22, 23) réalisés sous forme de substrat métallique, qui entoure la broche (8) respective qui est électriquement reliée au disque (20, 21, 22, 23) correspondant réalisé sous forme de substrat métallique, présente un diamètre inférieur à celui des autres trous (24).

13. Traversée filtrante selon la revendication 1, **caractérisée en ce qu'**on a prévu, sur le collet (5), une broche de raccordement supplémentaire (16) pour la connexion galvanique d'un dispositif de production et de traitement de signaux (14) de l'appareil de thérapie électronique, pouvant être implanté, à la paroi du boîtier (26).

14. Traversée filtrante selon la revendication 13, **caractérisée en ce que** la broche de raccordement (16) est réalisée pour l'essentiel sous forme de bande.

15. Appareil de thérapie électronique, pouvant être implanté, comportant un boîtier (26) et un dispositif électronique de production et de traitement de signaux (14) disposé dans ce boîtier (26), **caractérisé par** une traversée filtrante électrique unipolaire ou multipolaire (1) selon l'une quelconque des revendications précédentes destinée à relier le dispositif électronique de production et de traitement de signaux (14) à des lignes de transmission de signaux et de commande requises pour le fonctionnement de l'appareil de thérapie.
